# EUROPEAN PATENT APPLICATION

(11) **EP 4 129 434 A1**
(43) Date of publication of application: **08.02.2023**
(21) Application number: 21776954.6
(22) Date of filing: 19.03.2021
(51) Int. Cl.: B01D 11/00, A61K 36/886, A23L 19/00

(54) **SUPERCRITICAL FLUID EXRACTION DEVICE AND METHOD FOR MANUFACTURING TARGET PRODUCT USING SAME**

(30) Priority: 24.03.2020 JP 2020053253
(71) Applicant: Morinaga Milk Industry Co., Ltd., Minato-ku Tokyo 108-8384 (JP)
(72) Inventor: MOTOYOSHI, Tomomi, Zama-shi, Kanagawa 252-8583 (JP); SATO, Yoshiyasu, Zama-shi, Kanagawa 252-8583 (JP); OZAWA, Tomohito, Zama-shi, Kanagawa 252-8583 (JP)
(74) Representative: Hoffmann Eitle
(86) International application number: PCT/JP2021/011339
(87) International publication number: WO 2021/193418

(57) **Abstract**

An object of the present invention is to provide a supercritical fluid extraction apparatus capable of achieving high recovery efficiency even when a target is in a solid state or a semi-solid state, and a method for producing a target using the apparatus.

A supercritical fluid extraction apparatus 1 that produces a target p using a supercritical fluid F includes an extraction tank A in which a raw material P of the target p is accommodated and to which the supercritical fluid F is supplied, a separation tank B that separates the target p and the supercritical fluid F, and a recovery container C in which the target p is accommodated, in which the recovery container C is detachably disposed in the separation tank B.

## Description

### Technical Field

The present invention relates to an apparatus for separating and extracting a target using a supercritical fluid and a method for producing a target using the apparatus.

### Background Art

Conventionally, supercritical fluid extraction has been widely used as an extraction technique mainly for foods and pharmaceuticals.

Supercritical fluid extraction is an extraction technique for separating and extracting a target component depending on a difference in solubility by adding a supercritical fluid as an extraction medium to a raw material containing a target and bringing these into counterflow contact with each other.

In addition, since the solubility of the supercritical fluid changes by adjusting the pressure and temperature, it is possible to selectively extract the target.

As a technique using this supercritical fluid extraction, Patent Literature 1 describes an invention related to a supercritical fluid extraction system capable of efficiently recovering a target.

The supercritical fluid extraction system includes a back pressure adjusting unit, a temperature adjusting unit, and the like, and an object thereof is to efficiently recover a target by making the pressure and the temperature of the supercritical fluid adjustable within an optimum range for recovering the target using these units.

### Citation List

### Patent Literature

Patent Literature 1: JP 2007-120972 A

### Summary of Invention

### Technical Problem

Incidentally, the conventional apparatus used for supercritical fluid extraction including the invention described in Patent Literature 1 is used on the premise that a target is a liquid such as an essential oil, and the target usually passes through a pipe provided at the bottom of a separation tank and is recovered.

Here, when the target is in a solid state or a semi-solid state, depending on the size and the degree of viscosity of the target, the target does not pass through the pipe and cannot be recovered. Therefore, the target is recovered from the upper opening of the separation tank by using a recovery tool separately.

However, the separation tank is usually configured as a cylindrical container having a height of 1 m or more, and it is necessary to perform an operation of recovering a target accumulated on the bottom from the upper opening.

Therefore, when the target is in a solid state or a semi-solid state, there is a problem that the recovery efficiency is significantly deteriorated.

The present invention has been made in view of the above circumstances, and it is an object of the present invention to provide a supercritical fluid extraction apparatus capable of achieving high recovery efficiency even when the target is in a solid state or a semi-solid state, and a method for producing a target using the apparatus.

### Solution to Problem

In order to solve the above problems, the present invention provides a supercritical fluid extraction apparatus for producing a target using a supercritical fluid, the apparatus including:
an extraction tank in which a raw material of the target is accommodated and to which the supercritical fluid is supplied, a separation tank that separates the target and the supercritical fluid, and a recovery container in which the target is accommodated, wherein
the recovery container is detachably disposed in the separation tank.

According to the present invention, high recovery efficiency can be achieved even when the target is in a solid state or a semi-solid state.

That is, by providing the recovery container detachable from the separation tank, the operator can remove the recovery container from the separation tank and easily and reliably recover the target from the recovery container. As a result, the difficulty of the recovery operation caused by the structure such as the height of the separation tank is eliminated, and high recovery efficiency can be achieved.

In a preferred embodiment of the present invention, the recovery container is a bottomed cylindrical body having an opening on an upper side.

With such a configuration, the target separated and extracted in the separation tank can be easily and efficiently accommodated in the recovery container using gravity.

In a preferred embodiment of the present invention, the separation tank is a bottomed cylindrical body having an opening on an upper side, and the recovery container is configured to be slidable toward the opening of the separation tank.

With such a configuration, the recovery container can be easily taken out through the opening on an upper side of the separation tank while the separation tank has a simple configuration.

In a preferred embodiment of the present invention, the outer shape of the recovery container is formed along the shape of the inner wall surface of the separation tank.

With such a configuration, it is possible to stably dispose the recovery container in the separation tank while securing a large volume of the recovery container.

In a preferred embodiment of the present invention, the recovery container has a flange portion formed at an opening end peripheral edge of the recovery container and protruding toward an inner wall surface of the separation tank.

With such a configuration, it is possible to minimize the gap between the upper portion of the recovery container and the inner wall surface of the separation tank, and to suppress the situation where the target enters between the recovery container and the separation tank through the gap.

In a preferred embodiment of the present invention, the recovery container has a handle portion to which a recovery tool is attached.

With such a configuration, the operation of taking out the recovery container from the separation tank can be easily and quickly performed.

In a preferred embodiment of the present invention, the supercritical fluid is carbon dioxide.

With such a configuration, as compared with a case where the supercritical fluid is an organic solvent, the solvent removal/concentration operation after extraction is easy, the critical temperature is as low as about 30 degrees, and the supercritical fluid is harmless to the human body, so that the separation and extraction operation can be performed safely and efficiently.

In a preferred embodiment of the present invention, the target is an aloe extract containing aloe vera mesophyll as a raw material.

Further, the present invention is a method for producing a target using a supercritical fluid extraction apparatus that includes an extraction tank in which a raw material of the target is accommodated and to which the supercritical fluid is supplied, a separation tank which separates the target and the supercritical fluid, and a recovery container in which the target is accommodated, wherein the recovery container is detachably disposed in the separation tank, the method including the steps of:
accommodating a raw material of the target in the extraction tank;
supplying a supercritical fluid to the extraction tank to generate a mixture of the target and the supercritical fluid;
separating and extracting the target from the mixture in the separation tank and accommodating the target in the recovery container;
taking out the recovery container from the separation tank; and
recovering the target from the recovery container.

### Advantageous Effects of Invention

According to the present invention, it is possible to provide a supercritical fluid extraction apparatus capable of achieving high recovery efficiency even when a target is in a solid state or a semi-solid state, and a method for producing a target using the apparatus.

### Brief Description of Drawings

Fig. 1 is a schematic diagram of a supercritical fluid extraction system using a supercritical fluid extraction apparatus according to an embodiment of the present invention.
Fig. 2 is a diagram showing a recovery container according to an embodiment of the present invention, where (a) is a schematic perspective view and (b) is a cross-sectional view taken along a line XX '.
Fig. 3 is a longitudinal sectional view showing a recovery container and a separation tank according to an embodiment of the present invention.
Fig. 4 is a diagram for explaining a method for producing a target using the supercritical fluid extraction apparatus according to the embodiment of the present invention.
Fig. 5 is a diagram for explaining a method for producing a target using the supercritical fluid extraction apparatus according to the embodiment of the present invention.
Fig. 6 is a schematic perspective view illustrating a recovery container according to another embodiment of the present invention.
Fig. 7 is a diagram showing a recovery container according to another embodiment of the present invention, where (a) is a cross-sectional view taken along a line YY ', and (b) is a bottom view.
Fig. 8 is a longitudinal sectional view showing a recovery container and a separation tank according to another embodiment of the present invention.

### Description of Embodiments

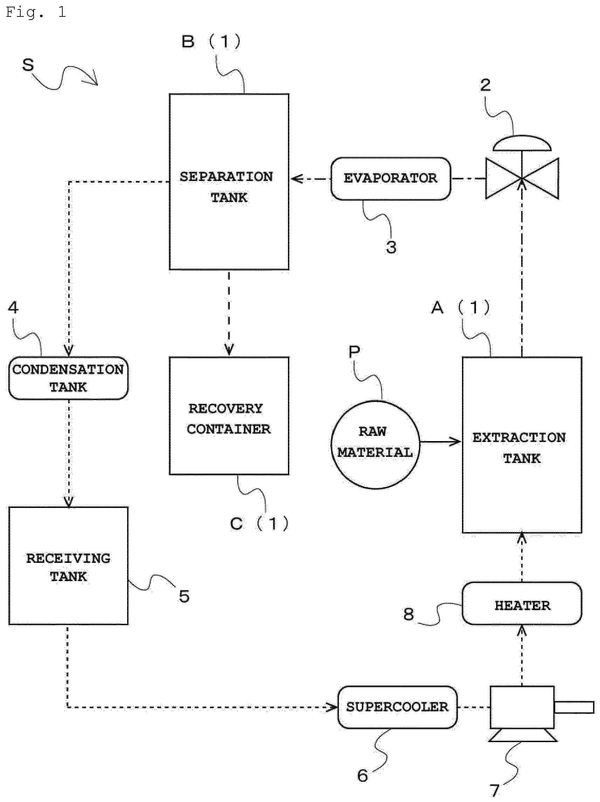

Hereinafter, a supercritical fluid extraction apparatus according to an embodiment of the present invention and a method for producing a target using the apparatus will be described with reference to Figs. 1 to 8.

Note that the following embodiments are examples of the present invention, and the present invention is not limited to the following embodiments. In these drawings, reference numeral 1 denotes a supercritical fluid extraction apparatus according to the present embodiment.

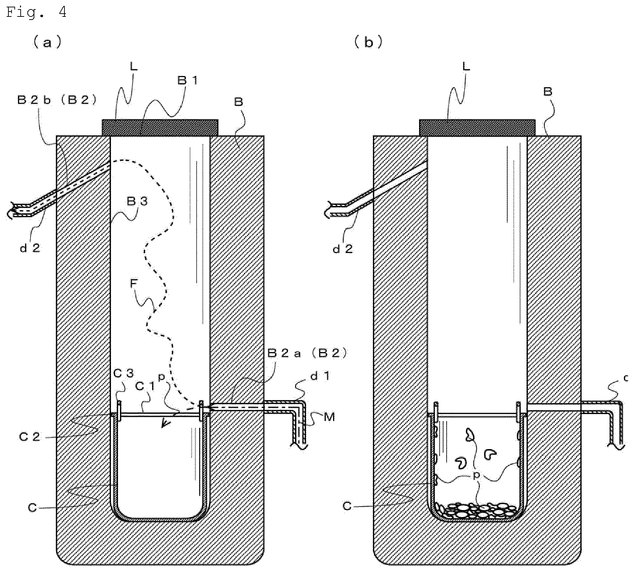

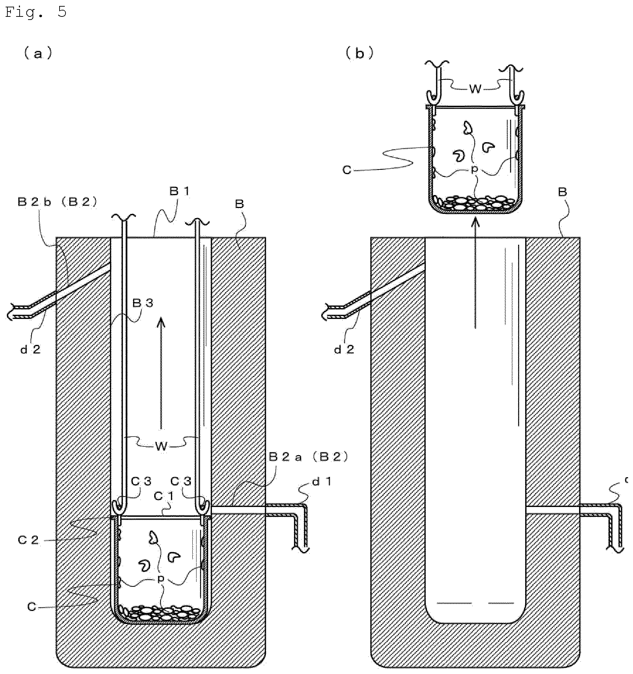

As shown in Fig. 1, the supercritical fluid extraction apparatus 1 includes an extraction tank A in which a raw material P of a target p (see Figs. 4 and 5) is accommodated and to which a supercritical fluid F (see Fig. 4) is supplied, a separation tank B that separates the target p and the supercritical fluid F, and a recovery container C in which the target p is accommodated.

Further, the supercritical fluid extraction system X includes, in addition to the supercritical fluid extraction apparatus 1, a pressure regulating valve 2, an evaporator 3, a condensation tank 4, a receiving tank 5, a supercooler 6, a circulation pump 7, and a heater 8.

The pressure regulating valve 2 regulates a pressure difference between the extraction tank A and the separation tank B in order to deliver a mixture M of the raw material P and the supercritical fluid F (see Fig. 4) from the extraction tank A to the separation tank B.

The evaporator 3 evaporates and vaporizes the mixture M.

The condensation tank 4 condenses and liquefies the supercritical fluid F separated in the separation tank B.

The receiving tank 5 stores the supercritical fluid F condensed by the condensation tank 4.

The supercooler 6 brings the supercritical fluid F stored in the receiving tank 5 into a supercooled state.

The circulation pump 7 feeds the supercritical fluid F stored in the receiving tank 5 to the extraction tank A via the supercooler 6 and the heater 8.

The heater 8 heats the supercritical fluid F brought into the supercooled state by the supercooler 6 to bring the supercritical fluid F into the supercritical state.

In the present embodiment, the respective components of the supercritical fluid extraction system X cooperate with each other to perform the operation of circulating the supercritical fluid F and separating and extracting the target p using the supercritical fluid F.

Note that, in Fig. 1, an arrow of a one-dot chain line indicates the flow of the mixture M, an arrow of a broken line indicates the flow of the target p, and an arrow of a dotted line indicates the flow of the supercritical fluid F.

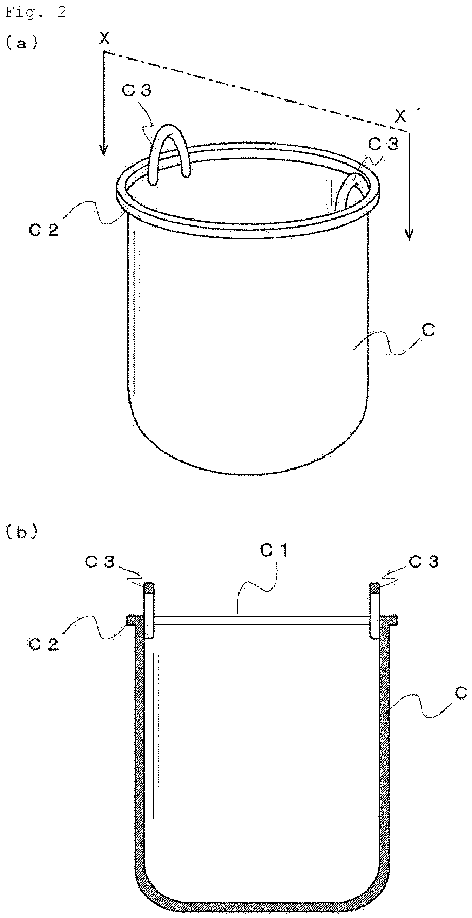

As shown in Fig. 2, the recovery container C is formed into a bottomed cylindrical body having an opening C1 on the upper side.

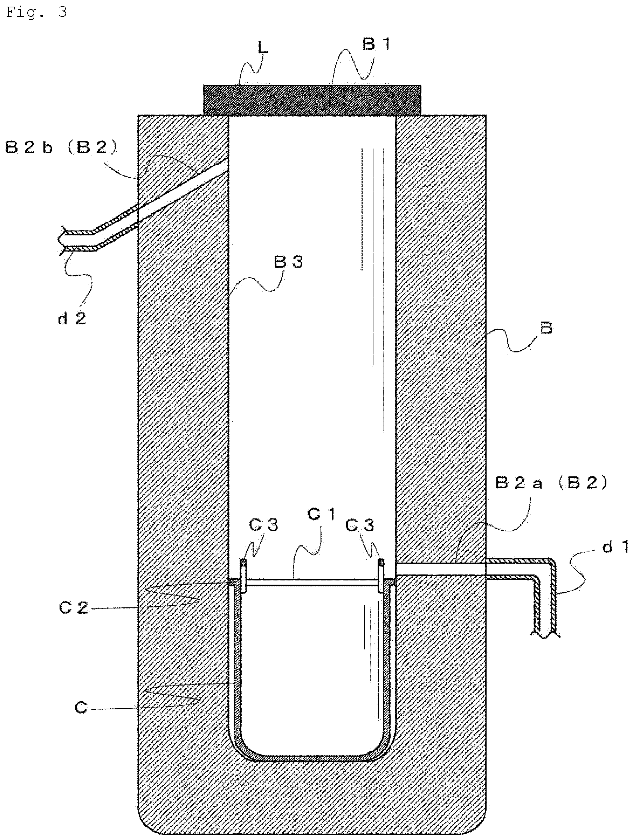

In addition, the recovery container C includes a flange portion C2 formed on an opening end peripheral edge thereof and protruding toward an inner wall surface B3 (see Fig. 3) of the separation tank B, and a pair of handle portions C3 to which the recovery tool W (see Fig. 5) is attached.

As a material of the recovery container C, for example, stainless steel is suitably used.

The pair of handle portions C3 is formed in a substantially inverted U shape, and is provided to face the upper side of the inner peripheral surface of the recovery container C so that the upper side of the handle portion C3 protrudes from the opening C1.

As shown in Fig. 3, the separation tank B is configured as a bottomed cylindrical body having an opening B1 on the upper side.

Further, the separation tank B has a communication hole B2 that allows the inside and the outside to communicate with each other.

The communication hole B2 includes a lower communication hole B2a provided below the separation tank B and an upper communication hole B2b provided above the separation tank B.

One end of a connection pipe d1 to which the evaporator 3 is connected at the other end is provided at the external opening end of the lower communication hole B2a, and the mixture M evaporated and vaporized by the evaporator 3 passes through the connection pipe d1 and the lower communication hole B2a and flows into the separation tank B.

One end of a connection pipe d2 to which the condensation tank 4 is connected at the other end is provided at the external opening end of the upper communication hole B2b, and the supercritical fluid F separated and extracted in the separation tank B passes through the upper communication hole B2b and the connection pipe d2 and flows into the condensation tank 4.

As shown in Fig. 3, the recovery container C is formed along the shape of the inner wall surface B3 of the separation tank B, and is disposed inside the separation tank B so that the bottom surface of the recovery container C is in contact with the inner bottom surface of the separation tank B.

Since the peripheral edge of the flange portion C2 of the recovery container C and the inner wall surface B3 of the separation tank B are arranged so as to form a slight gap, the recovery container C is configured to be slidable toward the opening B1 of the separation tank B.

That is, the recovery container C is detachably disposed inside the separation tank B.

Further, the recovery container C is configured so that the height from the outer bottom surface to the opening end thereof is lower than the height from the inner bottom surface of the separation tank B to the lower communication hole B2a. This prevents the outer peripheral surface of the recovery container C from closing the opening of the lower communication hole B2a.

Hereinafter, a method for producing the target p using the supercritical fluid extraction apparatus 1 will be described with reference to Figs. 4 and 5.

Here, in the description of the present embodiment, the raw material P is an aloe granulated product obtained from aloe vera mesophyll, the target p is a waxy aloe extract, and carbon dioxide is used as the supercritical fluid F. However, in the present invention, if the target p is in a solid state or a semisolid state, a raw material other than aloe mesophyll can be used as the raw material P, and a supercritical fluid other than carbon dioxide can also be used.

First, the operator accommodates the raw material P in the extraction tank A, and then further supplies the supercritical fluid F to the extraction tank A.

More specifically, the operator freeze-dries or hot-air-dries a mesophyll (transparent gel) portion of aloe vera not containing a leaf bark to prepare powdered aloe vera mesophyll, and accommodates the powdered aloe vera mesophyll as a raw material P in the extraction tank A.

The operator may produce an aloe granulated product by sufficiently mixing the prepared powdered aloe vera mesophyll and water, and accommodate the aloe granulated product in the extraction tank A as the raw material P.

As a result, a desired component of the raw material P is dissolved in the supercritical fluid F, and a mixture M of the target p and the supercritical fluid F is generated.

Note that, detailed illustration in the above steps is omitted. The aloe granulated product can be used as the raw material P as it is or after being dried.

Here, as the supercritical fluid F, supercritical propane, supercritical ethylene, supercritical 1,1,1,2-tetrafluoroethane, or the like can be used, but it is preferable to use carbon dioxide from the viewpoint of improving safety as food and drink.

The extraction temperature can be appropriately selected in a temperature range of 28°C to 120°C. However, the extraction temperature is preferably in a range of 50 to 69°C, and more preferably in a range of 50 to 59°C in order to improve the extraction efficiency of plant sterols that can be added to foods such as a cyclolanostane compound and a rophenol compound, and to reduce the content of anthraquinone-based compounds (for example, aloeemodin) having a laxative property.

The pressure inside the extraction tank A can be appropriately selected in the range of 5.5 to 60 MPa, but is preferably in the range of 15 to 60 MPa, and more preferably in the range of 15 to 24 MPa in order to improve the extraction efficiency of the cyclolanostane compound and the rophenol compound and to reduce the content of the anthraquinone-based compound.

In addition, it is also possible to use an entrainer such as ethanol from the viewpoint of improving the extraction efficiency of the cyclolanostane compound and the rophenol compound, but it is preferable not to use an entrainer from the viewpoint of reducing the content of the anthraquinone-based compound.

Next, as shown in Fig. 4(a), the operator causes the mixture M to flow into the separation tank B from the evaporator 3 through the connection pipe d1 and the lower communication hole B2a.

At this time, inside the connection pipe d1 and the lower communication hole B2a, the mixture M is substantially in a gaseous state, but the internal pressure of the separation tank B is set to be lower than the internal pressure of the extraction tank A by the pressure regulating valve 2, and thus the mixture M is separated into the target p and the supercritical fluid F at the time when the mixture M flows into the separation tank B.

As a result, the target p is accommodated in the recovery container C.

Note that, in Fig. 4(a), as in Fig. 1, the arrow of a one-dot chain line indicates the flow of the mixture M, the arrow of a broken line indicates the flow of the target p, and the arrow of a dotted line indicates the flow of the supercritical fluid F.

Then, by performing the separation and extraction operation described above on the entire amount of the mixture M accommodated in the extraction tank A, the target p is accumulated in the recovery container C as illustrated in Fig. 4(b).

Since the target p is a waxy aloe extract having a predetermined viscosity, the target p may adhere to the inner peripheral surface of the recovery container C as illustrated in Fig. 4(b). Further, the opening B1 of the separation tank B is closed by a lid portion L while the operation of separation extraction and the operation of accommodating the target p in the recovery container described above are completed.

Next, as illustrated in Fig. 5(a), the operator removes the lid portion L from the separation tank B, inserts the recovery tool W into the separation tank B, and attaches the recovery tool W to the handle portion C3.

More specifically, the recovery tool W is a pair of rod-like members whose tips are formed in a hook shape, and an operation device (not illustrated) that operates the recovery tool W in the vertical direction is connected to the other end of the recovery tool W.

Therefore, the operator inserts and locks each tip of the recovery tool W into each of the handle portions C3, and operates the recovery tool W upward by the operation device.

As a result, as shown in Fig. 5(b), the recovery container C is slid toward the opening B1 of the separation tank B and taken out to the outside of the separation tank B. When the target p adheres to the inner wall surface B3 of the separation tank B, the operator removes the target p from the inner wall surface B3 using a tool such as a spatula as appropriate before taking out the recovery container C from the separation tank B, and drops the target p into the recovery container C to recover the target p.

Finally, the operator uses a tool such as a spatula to scrape and recover the target p accumulated in the recovery container C.

In the target p obtained by the above method, the cyclolanostane compound and the rophenol compound contained in aloe vera mesophyll are concentrated.

The standard of the total amount of the cyclolanostane compound and the rophenol compound in the target p is preferably 0.5 mass% or more, more preferably 0.6 mass% or more, and particularly preferably 0.7 mass% or more. The upper limit value of the total amount is generally 1 mass%.

In addition, the obtained target p can be used as an active ingredient for food and drink, cosmetics, medicine, and the like as it is or through further operations such as extraction and drying.

For example, a medicine containing the target p of the present invention can be appropriately produced by combining the target p of the present invention with a pharmaceutically acceptable formulation carrier or the like, and in formulation, additives commonly used as formulation carriers such as excipients, binders, disintegrants, lubricants, stabilizers, flavoring agents, diluents, surfactants, and injectable solvents can be used.

Here, in the production method (a) using the recovery container C of the present invention, the production method (b) not using the recovery container C, and the production method (c) using a bucket instead of the recovery container C, tables comparing the recovery efficiency of the cyclolanostane compound and the recovery efficiency of the rophenol compound in the target p are shown below.

Here, the recovery efficiency of the cyclolanostane compound and the rophenol compound refers to the ratio of the amounts of the cyclolanostane compound and the rophenol compound in the recovered target p to the amounts of the cyclolanostane compound and the rophenol compound in the raw material P, expressed in 100 fractions.

Incidentally, a bucket which is generally commercially available and has a bottomed cylindrical shape, an outer diameter gradually decreasing toward a bottom portion, and an inverted U-shaped handle provided at an upper portion is used.

**[Table 1]**

| | Amount of cyclolanostane compound and rophenol compound in raw material P | Amount of cyclolanostane compound and rophenol compound in recovered target p | Recovery efficiency of cyclolanostane compound and rophenol compound |
|---|---|---|---|
| (a)With recovery container C | 19.51 g | 12.71 g | 65.15% |
| (b)Without recovery container C | 12.84 g | 5.90 g | 46.00% |
| (c)Buckets | 10.39 g | 5.90 g | 56.79% |

As shown in Table 1, according to the production method (a) using the recovery container C of the present invention which is detachably disposed in the separation tank B and has an outer shape formed along the inner wall surface of the separation tank B, high recovery efficiency of the cyclolanostane compound and the rophenol compound in the target p can be achieved as compared with other methods.

In the production method (b) not using the recovery container C, it is difficult to recover the target p at the bottom of the separation tank B. Therefore, it can be seen that the recovery efficiency of the cyclolanostane compound and the rophenol compound is significantly lower than that in (a).

In addition, in the production method (c) using a bucket instead of the recovery container C, although the target p at the bottom of the separation tank B can be recovered, the target p that has entered the gap between the outer peripheral surface of the bucket and the inner wall surface of the separation tank B cannot be sufficiently recovered. Therefore, it can be also seen that the recovery efficiency of the cyclolanostane compound and the rophenol compound is lower than that in (a).

According to the present embodiment, the operator can remove the recovery container C from the separation tank B and easily and reliably recover the target p from inside the recovery container C. As a result, the difficulty of the recovery operation caused by the structure such as the height of the separation tank B is eliminated, and high recovery efficiency can be achieved.

In addition, since the recovery container C is the bottomed cylindrical body having the opening C1 on the upper side, the target separated and extracted in the separation tank B can be easily and efficiently accommodated in the recovery container C using gravity.

In addition, since the separation tank B is the bottomed cylindrical body having the opening B1 on the upper side, and the recovery container C is configured to be slidable toward the opening B1 of the separation tank B, the recovery container C can be easily taken out through the opening B1 above the separation tank B while the separation tank B has a simple configuration.

In addition, since the outer shape of the recovery container C is formed along the shape of the inner wall surface of the separation tank B, it is possible to stably dispose the recovery container C inside the separation tank B while securing a large volume of the recovery container C.

In addition, since the recovery container C has the flange portion C2, it is possible to minimize the gap between the upper portion of the recovery container C and the inner wall surface B3 of the separation tank B, and to suppress the situation in which the target p enters between the recovery container C and the separation tank B through the gap.

In addition, since the recovery container C has the handle portion C3 to which the recovery tool W is attached, it is possible to easily and quickly take out the recovery container C from the separation tank B.

In addition, since the supercritical fluid F is carbon dioxide, it is possible to safely and efficiently perform the separation and extraction operation.

Note that the shapes, dimensions, and the like of the constituent members described in the above embodiments are merely examples, and can be variously changed on the basis of design requirements and the like.

For example, in the above embodiment, the height of the recovery container C is set so that the outer peripheral surface of the recovery container C does not block the opening of the lower communication hole B2a, but the height of the recovery container C may be further increased from the above embodiment.

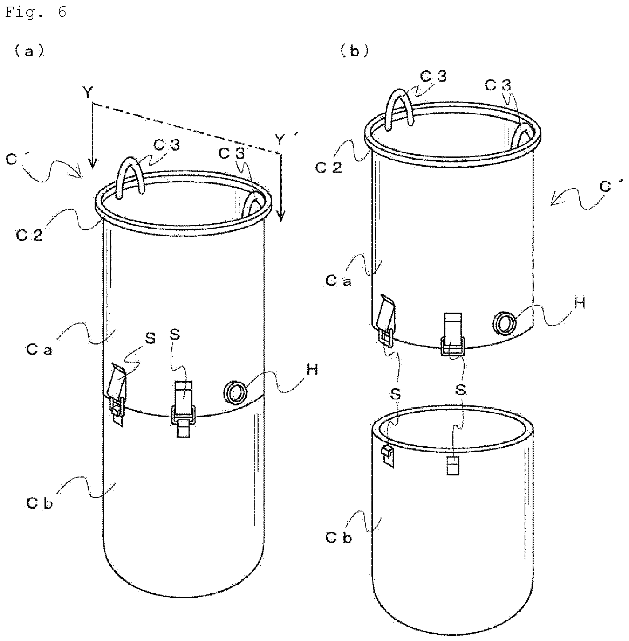

In this case, preferable configurations of a recovery container C' and a separation tank B' will be described as another embodiment with reference to Figs. 6 to 8.

As shown in Fig. 6, the recovery container C' is formed of a first recovery container constituent body Ca and a second recovery container constituent body Cb which are coupled so as to be dividable in the vertical direction.

More specifically, as shown in Fig. 6(a), the first recovery container constituent body Ca and the second recovery container constituent body Cb are connected by a plurality of coupling means S provided on the outer peripheral surface thereof to form one bottomed cylindrical body.

Note that the protruding length of the plurality of coupling means S from the outer peripheral surface of the first recovery container constituent body Ca is set shorter than the protruding length of the flange portion C2 from the outer peripheral surface of the first recovery container constituent body Ca. Further, the plurality of coupling means S in the present embodiment are so-called snap locks, but are not limited thereto, and may be any means as long as the first recovery container constituent body Ca and the second recovery container constituent body Cb can be freely divided and coupled.

Further, as shown in Fig. 6(b), by releasing the engagement state of the plurality of coupling means S, the first recovery container constituent body Ca and the second recovery container constituent body Cb can be easily divided.

Similarly to the recovery container C, the first recovery container constituent body Ca includes a flange portion C2 protruding toward the inner wall surface B3 of the separation tank B and a pair of handle portions C3 to which the recovery tool W is attached.

In addition, the first recovery container constituent body Ca has a through hole H on the outer peripheral surface thereof.

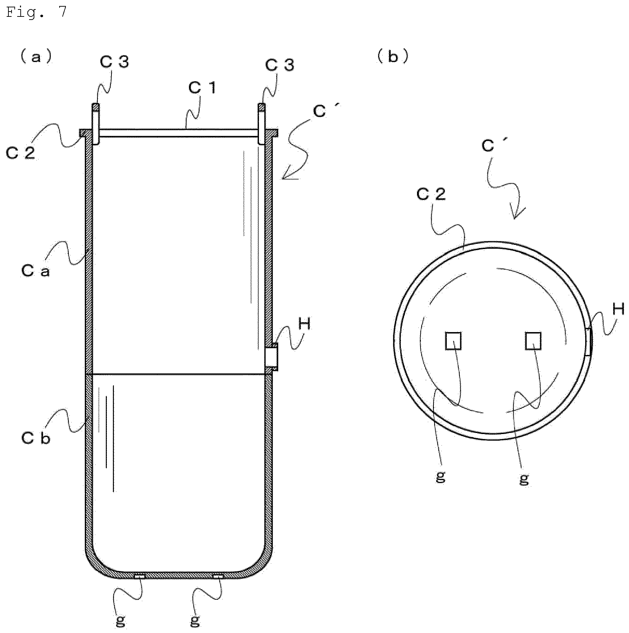

As illustrated in Fig. 7, the second recovery container constituent body Cb has two fitting holes g in an outer bottom surface thereof.

The protruding length of the through hole H from the outer peripheral surface of the first recovery container constituent body Ca is substantially the same as the protruding length of the flange portion C2 from the outer peripheral surface of the first recovery container constituent body Ca.

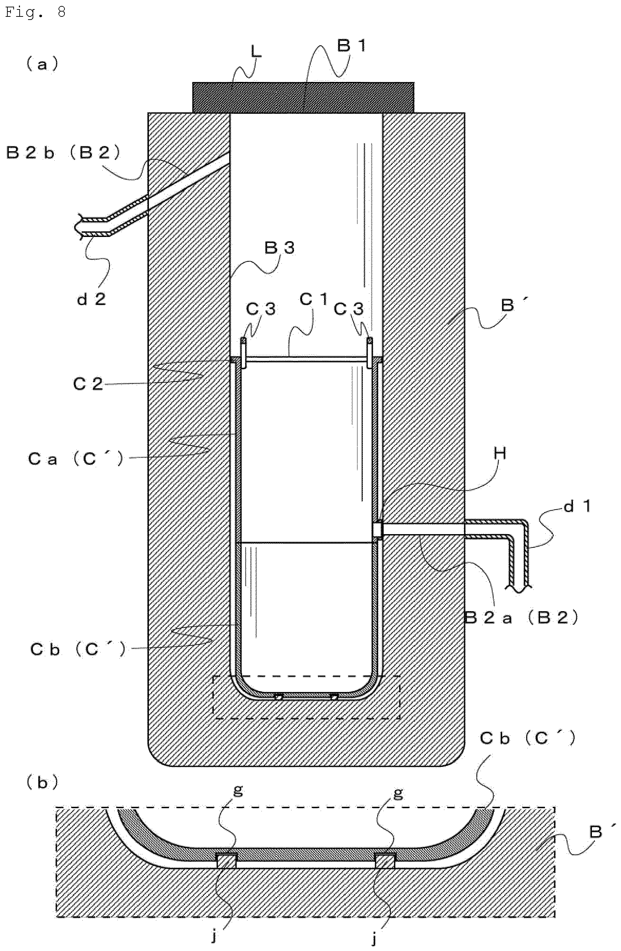

Fig. 8 is a longitudinal sectional view when the recovery container C' configured as described above is disposed inside the separation tank B'.

Fig. 8(b) is an enlarged view of a dotted frame portion in Fig. 8(a).

As shown in Fig. 8, the separation tank B' has two fitting protrusions j fitted into the respective fitting holes g on the inner bottom surface thereof.

That is, positioning means is configured by the fitting holes g and the fitting protrusions j, and the rotation operation of the recovery container C' inside the separation tank B' is restricted by the positioning means.

Note that, since the separation tank B' has the same configuration as the separation tank B except for the configuration having the fitting protrusions j, the description thereof will be omitted. Further, the configuration of the positioning means is not necessarily the fitting hole g and the fitting protrusion j, and is not particularly limited as long as the rotation operation of the recovery container C' is restricted.

The height from the outer bottom surface of the recovery container C' to the through hole H is substantially the same as the height from the inner bottom surface of the separation tank B' to the lower communication hole B2a. As a result, the inside of the recovery container C' and the lower communication hole B2a communicate with each other via the through hole H, and the mixture M reliably flows into the separation tank B'.

Since the flow of the operation of taking out the recovery container C' using the recovery tool W is similar to that of the previous embodiment, the description thereof will be omitted.

According to the present embodiment, it is possible to arbitrarily increase the volume of the recovery container C' without restricting the height of the recovery container C', and it is possible to suppress a situation in which the recovery operation becomes troublesome due to the attachment of the target p to the inner peripheral surface of the separation tank B'.

In addition, the fitting hole g and the fitting protrusion j prevent the outer peripheral surface of the recovery container C' from closing the opening of the lower communication hole B2a due to unexpected rotation of the recovery container C' or the like.

In addition, since the recovery container C' includes the first recovery container constituent body Ca and the second recovery container constituent body Cb that can be divided, the target p accumulated at the bottom of the second recovery container constituent body Cb can be easily recovered.

In each of the above embodiments, an example in which the target p is an aloe extract that is waxy has been described. However, the supercritical fluid extraction apparatus 1 of each embodiment can be naturally applied also to a case where the target p is a liquid or in another solid state or semi-solid state.

### Reference Signs List

- 1: supercritical fluid extraction apparatus
- A: extraction tank
- B, B': separation tank
- B1: opening
- B2: communication hole
- B2a: lower communication hole
- B2b: upper communication hole
- B3: Inner wall surface
- j: fitting protrusion
- C, C': recovery container
- C1: opening
- C2: flange portion
- C3: handle portion
- Ca: first recovery container constituent body
- Cb: second recovery container constituent body
- H: through hole
- S: coupling means
- g: fitting hole
- d1, d2: connection pipe
- L: lid portion
- X: supercritical fluid extraction system
- 2: pressure regulating valve
- 3: evaporator
- 4: condensation tank
- 5: receiving tank
- 6: supercooler
- 7: circulation pump
- 8: heater
- P: raw material
- p: target
- F: supercritical fluid
- M: mixture

## Claims

1. A supercritical fluid extraction apparatus for producing a target using a supercritical fluid, the apparatus comprising:
an extraction tank in which a raw material of the target is accommodated and to which the supercritical fluid is supplied, a separation tank which separates the target and the supercritical fluid, and a recovery container in which the target is accommodated, wherein
the recovery container is detachably disposed in the separation tank.

2. The supercritical fluid extraction apparatus according to claim 1, wherein the recovery container is a bottomed cylindrical body having an opening on an upper side.

3. The supercritical fluid extraction apparatus according to claim 2, wherein
the separation tank is a bottomed cylindrical body having an opening on an upper side, and
the recovery container is configured to be slidable toward an opening of the separation tank.

4. The supercritical fluid extraction apparatus according to any one of claims 1 to 3, wherein an outer shape of the recovery container is formed along a shape of an inner wall surface of the separation tank.

5. The supercritical fluid extraction apparatus according to claim 4, wherein the recovery container has a flange portion formed at an opening end peripheral edge of the recovery container and protruding toward an inner wall surface of the separation tank.

6. The supercritical fluid extraction apparatus according to any one of claims 1 to 5, wherein the recovery container has a handle portion to which a recovery tool is attached.

7. The supercritical fluid extraction apparatus according to any of claims 1 to 6, wherein the supercritical fluid is carbon dioxide.

8. The supercritical fluid extraction apparatus according to any one of claims 1 to 7, wherein the target is an aloe extract containing aloe vera mesophyll as a raw material.

9. A method for producing a target using a supercritical fluid extraction apparatus that includes an extraction tank in which a raw material of the target is accommodated and to which a supercritical fluid is supplied, a separation tank which separates the target and the supercritical fluid, and a recovery container in which the target is accommodated, wherein
the recovery container is detachably disposed in the separation tank, the method comprising the steps of:
accommodating a raw material of the target in the extraction tank;
supplying a supercritical fluid to the extraction tank to generate a mixture of the target and the supercritical fluid;
separating and extracting the target from the mixture in the separation tank and accommodating the target in the recovery container;
taking out the recovery container from the separation tank; and
recovering the target from the recovery container.
